# EUROPEAN PATENT APPLICATION

(11) **EP 2 415 874 A1**
(43) Date of publication of application: **08.02.2012**
(21) Application number: 10757986.4
(22) Date of filing: 14.01.2010
(51) Int. Cl.: C12Q 1/04, C12M 1/16

(54) **METHOD FOR TESTING DRUG SENSITIVITY AND DEVICE THEREOF**

(30) Priority: 01.04.2009 CN 200910043007
(71) Applicant: Hunan-Tech New Medical Systems Co., Ltd, Hunan 410000 (CN)
(72) Inventor: PENG, Jun, Hunan 410000 (CN)
(74) Representative: Hryszkiewicz, Danuta
(86) International application number: PCT/CN2010/000064
(87) International publication number: WO 2010/111883

(57) **Abstract**

The present invention discloses a method for testing drug sensitivity and device thereof. The method comprises the steps of: preparing liquid samples by adding the medium containing gel materials to the concentrated bacterium or cell samples, adding coagulants to the liquid samples to form the colloidal samples, placing the drug test paper on the surface of the colloidal samples, and determining the drug sensitivity by observing the drug inhibition zone after cultivation. The device for testing drug sensitivity is a box made of transparent materials and comprises coagulants in its cavity.

## Description

The invention relates to a method for testing drug sensitivity of bacteria or cells and a device used therefor.

Drug sensitivity test is very important for testing drug resistance of bacteria and cells. Conventional methods for drug sensitivity test include paper examination method and concentration examination method, and the culture medium involved therein includes a liquid medium and a solid medium. Generally, paper examination method employs a solid medium and concentration examination method employs a liquid medium. For paper examination method, a drug paper is pasted to a solid medium where bacteria are cultured, and then a drug inhibition zone forms. The size of the drug inhibition zone is decided by the sensitivity of the bacteria against the drug. For concentration examination method, the growth status of bacteria or cells cultured in containers with different drug concentrations is observed and the results therefrom are used for determining the sensitivity of the bacteria against the drug. The drug sensitivity test process, either using paper examination method or using concentration examination method, generally include three steps, i.e., concentrating bacteria or cells, multiplying or separating and culturing the bacteria or cells, and testing the drug sensitivity of the bacteria or cells. The following, taking tubercle bacillus as an example, describe the drug sensitivity test process using conventional methods. The process includes the above-mentioned three steps. Using paper examination method, the steps of concentrating tubercle bacillus include a) sucking a tubercle bacillus sample collected from a patient and placing the sample into a test container; b) adding a digestive juice into the container to digest the mucus of the sample so that tubercle bacillus is exposed; c) separating the sample using centrifugation and collecting the tubercle bacillus. Subsequently, the tubercle bacillus is multiplied or cultured. The steps of multiplying or separating and culturing the tubercle bacillus include d) collecting part of the concentrated tubercle bacillus and preparing it into a concentrated solution; e) inoculating the concentrated solution into a solid medium, for example, Roche medium, and culturing at 37°C for about a month, so that a single tubercle bacillus grows into a colony. The colony is further used for drug sensitivity test. The steps of testing the drug sensitivity of the tubercle bacillus include f) collecting a few of the colonies, generally 1-3 colonies, and dissolving and dispersing to yield a concentrated solution; g) inoculating the solution into the surface of a solid medium, such as agar medium; h) pasting drug paper on the surface of the solid medium; and i) placing the medium into an incubator, culturing at 37°C for about a month, and observing the formation of a drug inhibition zone. The size of the drug inhibition zone is decided by the sensitivity of the tubercle bacillus against the drug. Paper examination method is easy for practice, with simple equipment and low cost. However, the whole examination process takes a long time. The multiplying or separating and culturing the tubercle bacillus as well as testing the drug sensitivity separately take about a month, i.e., the whole process takes 2 months. If administering the drug after the test result is determined, good treatment timing may lose. Nevertheless, if administering the drug before the rest result is determined, a serious treatment consequence may occur. Furthermore, paper examination method is not safe because the process involves two times of artificial inoculation and tubercle bacillus is infectious. Thus, the method causes hidden danger to operators and environment.

Conventional concentration examination methods include two different types. One is that, the bacteria or cells are cultured in a solid medium and the steps of concentrating and multiplying or culturing bacteria or cells are the same as those for paper examination method. However, the steps of testing the drug sensitivity are different. Specifically, one or several bacteria colonies are dissolved and dispersed to yield a suspension. The suspension is put into containers with different drug concentrations respectively, and thus various test samples for comparison are obtained. The growth status of bacteria or cells cultured in the containers is observed and the results therefrom are used for determining the sensitivity of the bacteria against the drug. Equipment used in the method is simple and the cost is low. However, the whole examination process takes a long time. As mentioned above, the multiplying or separating and culturing the tubercle bacillus are the same as those for paper examination method, so it takes about a month. Furthermore, the method involves a plurality of test containers which is bothersome for preparation and may cause hidden danger to operators and environment. During the preparation, the test containers are easily contaminated by other bacteria, which causes false test results. The other concentration examination method employs a liquid medium during the steps of multiplying or culturing, and when testing the drug sensitivity, BACTEC 9000MB and BACTEC MGIT 960 (manufactured by BD company USA), an automatic rapid determination system of drug susceptibility of mycobacterium, is employed. The method is easy for practice and takes a short time. The average determination time is 14.4 days, the fastest, 10 days. The method, however, involves expensive equipment, a plurality of test containers, and expensive chemical agents, which means a high cost and a big difficulty to popularize in common hospitals.

There are many methods for cells in vitro to carry out drug sensitivity test, for example, MTT assay and radioisotope incorporation. These methods are complicated in practice and false test results may occur. The reasons lie in that, a plurality of test containers with different concentrations need preparing, and cell suspensions are added to the containers, both of which are bothersome. And during preparing the test containers, contamination may occur, thereby resulting in false test results.

Thus, based on conventional methods, the drug sensitivity test of tubercle bacillus takes a long time and has complicated operation and high cost, the process is not safe and even results in false test results.

Objectives of the invention are to provide a method for testing drug sensitivity of bacteria or cells and a device used therefor. The method and device used therefor are characterized by short testing time, low cost, and safe operation.

To achieve the above objectives, in accordance with one embodiment of the invention, there is provided a method for testing drug sensitivity of a bacterium or cell, comprising the steps of:

a) concentrating a bacterium or cell sample to be tested;

b) adding a culture solution to the bacterium or cell sample to yield a liquid sample, the culture solution comprising a nutrient and a colloidal material;

c) adding a coagulant aid to the liquid sample where the coagulant aid reacts with the colloidal material to yield a gel test sample;

d) pasting drug paper on the surface of the gel test sample;

e) culturing the gel test sample in an incubator; and

f) observing the formation of a drug inhibition zone and determining the drug sensitivity of the bacterium or cell.

The culture solution further comprises a growth indicator of the bacterium or cell.

The gel test sample is cultured in the incubator for 2-4 days.

The culture solution further comprises a bacteriostat.

For each 100 mL of the nutrient, the culture solution comprises 0.8-2 g of the colloidal material and the coagulant aid added thereto is 0.1-0.2 g.

For each 100 mL of the nutrient, the culture solution comprises 0.8-2 g of the colloidal material, the growth indicator of the bacterium or cell added thereto is 2-20 mg, and the coagulant aid 0.1-0.2 g.

For each 100 mL of the nutrient, the culture solution comprises 0.8-2 g of the colloidal material, the growth indicator of the bacterium or cell added thereto is 2-20 mg, appropriate bacteriostat, and the coagulant aid 0.1-0.2 g.

The culture solution is packed and sealed according to a one-time usage amount.

In another respect, the invention provides a device for testing drug sensitivity of bacterium or cell, the device comprising a box whose side walls and bottom are enclosed and whose top is open, the box comprising a chamber where a coagulant aid is disposed.

A coagulant aid layer is disposed at the bottom of the chamber of the box, comprising a slow-release carrier and the coagulant aid adhered thereto.

The coagulant aid layer is disposed at the bottom of the chamber of the box, comprising a mixture of an adhesive and the coagulant aid coated on the bottom of the box.

A scale is disposed at the bottom of the box.

A box cover is disposed to cooperate with the open top of the box.

The nutrient comprises a nutritional component and a buffer, providing nutrition for the growth of the bacteria or cells and controlling the pH value of the culture solution. For different categories of bacteria or cells, the nutrient has different formula accordingly. For example, middle brook 7H9 broth for tubercle bacillus, nutrient broth for common bacteria or fungi, and cell culture medium for cancer cells. The colloidal material, selected from the group consisting of 0.5-1.5% sodium alginate, 0.5-2.0% pectin, and 0.5-1.5% carrageenan, is dissolved in the culture solution to make it a liquid state prior to the addition of the coagulant aid. After the coagulant aid added, the colloidal material reacts with it to yield a stable gel in a short time. The growth indicator of bacteria or cells, preferably,
3-(4,5-dimethylthiazol-2-yl)-2,5-diphenyltetrazolium bromide (MTT), is degraded during the growth and reproduction of the bacteria and cells to produce a blue dot chromophoric group. That is to say, a visible blue dot forms in each bacterium or cell in a short time. The growth status of the bacterium or cell can be determined by observing quality and quantity of the blue dot with naked eye or a low-powered microscope. The bacteriostat is added, mainly during testing the drug sensitivity of tubercle bacillus, to prevent the growth of other bacteria other than acid-fastbacilli.

The coagulant aid, selected from the group consisting of calcium or magnesium ions, boric acid, and potassium citrate, reacts with the colloidal material of the culture solution to yield a stable gel.

The device of the invention, used for testing drug sensitivity of bacteria or cells, comprises the box whose side walls and bottom are enclosed and whose top is open, and the coagulant aid is disposed in the box. The culture solution comprises the colloidal material and is used for preparing a liquid sample to be tested. Thus, the liquid sample comprises the colloidal material. Before put into the device, the sample is liquid. After put into the device, the colloidal material of the sample reacts with the coagulant aid in the device and thus a gel test sample is produced quickly at normal temperature. Subsequently, drug paper is pasted on the surface of the gel test sample to determine the drug sensitivity of the bacteria or cells.

In contrast to conventional methods for testing drug sensitivity of bacteria or cells, the method of the invention takes shorter time. Here are the reasons. First, most of the to-be-tested bacteria or cells collected from a patient are concentrated to prepare a test sample and the entire test sample is used for drug sensitivity test, thus, the bacteria or cells are fully employed. Second, the time for multiplying or culturing the bacteria or cells is saved or decreased. Generally, six pieces of drug paper are enough for drug sensitivity test for common bacteria or cells. Thus, the chamber area of the device is designed large enough to accommodate the six pieces of drug paper simultaneously. The drug sensitivity test is used to test the drug resistance of a target bacterium or cell, so, prior to the test, the disease of a patient is often confirmed. For a patient whose disease is confirmed, because the bacteria or cells collected therefrom are fully employed, the quantity thereof in the chamber of the device is large enough to satisfy the requirement for drug sensitivity test. Consequently, the bacteria or cells don't need to be multiplied. If the bacteria are not enough for drug sensitivity test, the liquid sample prepared by the bacteria needs to be cultured for multiplication, followed by drug sensitivity test in the device. The culture is carried out in a liquid medium. Generally, the multiplication rate of the bacteria is much higher in a liquid medium than that in a solid medium. Furthermore, the original to-be-tested bacteria from the patient are large in quantity, so the culture time is very short. Take tubercle bacillus as an example, China's TB Control Program-sputum smear microscopy laboratory quality assurance manual (edited by Chinese Center for Disease Control and Prevention and published by Peking Union Medical College Press, Jul. 1, 2004) stipulates that, using Ziehl-Neelsen staining method, if the positive of acid-fast bacilli (4+) is greater than or equal to 10 per field of view using microscopic examination, the sample can be directly used for drug sensitivity test without culturing. Thus, the culture time is saved, so is the whole testing time. If the positive of acid-fast bacilli (3+) is less than or equal to 9 per field of view using microscopic examination, the sample need to be cultured. The culture carried out in the liquid medium has high multiplication rate, and the original bacteria from the patient are large in quantity, so the culture time is very short. Third, because the original bacteria are large in quantity, the drug inhibition zone forms quickly. Thus, the time for testing drug sensitivity is short, so is the whole testing time. The growth indicator added to the culture solution is conducive to observing the growth status of the bacteria or cells as well as the drug inhibition zone, which further saves the testing time. Studies show that, the method of the invention saves the steps of multiplication and culture, even multiplication and culture are needed, 2-4 days are enough. The steps of testing drug sensitivity take only 5-7 days. Thus, the whole testing process takes less than 14 days, which means the time is greatly saved.

The equipment involved in the invention is basically the same as that in conventional paper examination methods, i.e., a centrifugation device and a test device. The devices are easy to produce, with a low cost. Thus, the investment for the whole equipment is low. The disposable test containers involved in the invention and chemical agents, such as nutrients, buffers, colloidal materials, indicators, and so on, are very cheap and the consumption is small, which further reduces the cost. Consequently, the method of the invention has a low cost and low investment.

The concentration of a target sample and the observation of the results are achieved in a disposable sealed container, which prevents the contamination onto other equipment. The culture solution of the invention is stored in a sealed bottle. In use, the bottle is opened and the culture solution therein is mixed with the concentrated bacteria or cells. Subsequently, the mixture is pumped into the drug sensitivity test device. Thus, the manual operation involved in the method is few and very simple, which greatly reduces the risks caused by bacteria on operators or environment and improves the biological safety of the test.

The invention is described hereinbelow with reference to accompanying drawings, in which:

FIG. 1 is a sectional view of a drug sensitivity test device according to one embodiment of the invention; and

FIG. 2 is a bottom view of FIG. 1.

1. Box; 2. Coagulant aid; 3. Box cover; 4. Scale.

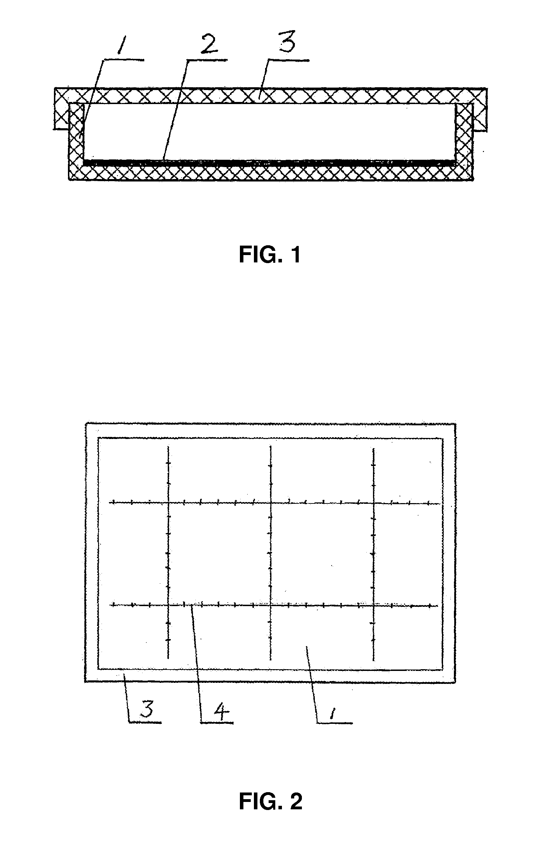

As shown in FIGS.1 and 2, a box 1 comprises enclosed side walls and bottom and an open top. A coagulant aid layer 2 is disposed inside the box. The coagulant aid layer 2 comprises a slow-release carrier and a coagulant aid adhered thereto. A scale 4 is disposed at the bottom of the box 1, and a box cover 3 is disposed to cooperate with the open top of the box 1.

The slow-release carrier of the coagulant aid layer 2 is a slow-release membrane. When a culture solution is dumped into the box 1, the slow-release membrane is dissolved therein and the coagulant aid adheres to the membrane. Optionally, the coagulant aid is mixed with a carbohydrate and then the resultant mixture is sprayed onto the box 1 to form the coagulant aid layer.

The following, taking tubercle bacillus as an example, describes a method of drug sensitivity test and a device used therefor.

Using Ziehl-Neelsen staining method, microscopic examination showed the positive of acid-fast bacilli (4+) was greater than or equal to 10 per field of view. The drug sensitivity test was carried out as follows:

a) collecting 5 mL of morning sputum sample from a patient and digesting the sample with 4% NaOH;

b) centrifuging the sample for 10 min at 4,500 rpm to precipitate tubercle bacillus therein;

c) removing a supernatant of the sample;

d) eluting a precipitate obtained from c) with 20 mL of a culture solution comprising a colloidal material to yield a liquid test sample;

e) dumping the liquid test sample evenly into a box of a drug sensitivity test device comprising a coagulant aid, the colloidal material of the culture solution reacting with the coagulant aid to yield a stable gel within 3 hrs, thereby a gel test sample obtained;

f) pasting paper comprising to-be-tested drug on the surface of the gel test sample;

g) sealing the box cover using a box cover and culturing in an incubator at 37°C for 5-7 days, during the growth and multiplication of tubercle bacillus, MTT being degraded to yield a blue dot chromophoric group, if the drug inhibiting the growth and multiplication of tubercle bacillus, no blue dot chromophoric group formed around the gel test sample pasted with paper comprising the drug, and an inhibition zone formed instead; and

h) observing the size of the inhibition zone and determining the effect of the drug on the bacteria.

A formula of 20 mL of the culture solution comprised 20 mL of middle brook 7H9 broth, 0.4 mg of 3-(4,5-dimethylthiazol-2-yl)-2,5-diphenyltetrazolium bromide as an indicator, 0.17 g of 0.5-1.5% sodium alginate as the colloidal material, 0.4 mg of malachite green as a bacteriostat. The coagulant aid was 0.02 g of calcium ion.

Optionally, a formula of 20 mL of the culture solution comprised 20 mL of middle brook 7H9 broth, 0.5 mg of
3-(4,5-dimethylthiazol-2-yl)-2,5-diphenyltetrazolium bromide as an indicator, 0.3 g of 0.5-1.5% sodium alginate as the colloidal material, 0.4 mg of malachite green as a bacteriostat (0.8 mL). The coagulant aid was 0.03 g of calcium ion.

Optionally, a formula of 20 mL of the culture solution comprised 20 mL of middle brook 7H9 broth, 4 mg of
3-(4,5-dimethylthiazol-2-yl)-2,5-diphenyltetrazolium bromide as an indicator, 0.4 g of the colloidal material, 0.4 mg of malachite green as a bacteriostat (0.8 mL). The coagulant aid was 0.04 g of calcium ion.

Using Ziehl-Neelsen staining method, microscopic examination showed the positive of acid-fast bacilli (3+) was less than 9 per field of view. The drug sensitivity test was carried out as follows:

a) collecting 5 mL of morning sputum sample from a patient and digesting the sample with 4% NaOH;

b) centrifuging the sample for 10 min at 4,500 rpm to precipitate tubercle bacillus of the sample;

c) removing a supernatant of the sample;

d) eluting a precipitate obtained from c) with 20 mL of a culture solution comprising a colloidal material to yield a liquid test sample;

e) culturing the liquid test sample in an incubator at 37°C for 2-4 days and observing the sample to turn into light blue;

f) dumping the cultured liquid test sample evenly into a box of a drug sensitivity test device comprising a coagulant aid, the colloidal material of the culture solution reacting with the coagulant aid to yield a stable gel within 3 hrs, thereby a gel test sample obtained;

g) pasting paper comprising to-be-tested drug on the surface of the gel test sample;

h) sealing the box cover using a box cover and culturing in an incubator at 37°C for 5-7 days, during growth and multiplication of tubercle bacillus, MTT being degraded to yield a blue dot chromophoric group, if the drug inhibiting the growth and multiplication of tubercle bacillus, no blue dot chromophoric group formed around the gel test sample pasted with paper comprising the drug, and an inhibition zone formed instead; and

i) observing the size of the inhibition zone and determining the effect of the drug on the bacteria.

A formula of the 20 mL of the culture solution comprised 20 mL of middle brook 7H9 broth, 0.5 mg of
3-(4,5-dimethylthiazol-2-yl)-2,5-diphenyltetrazolium bromide as an indicator, 0.3 g of 0.5-1.5% sodium alginate as the colloidal material, 0.4 mg of malachite green as a bacteriostat. The coagulant aid was 0.03 g of calcium ion.

The following, taking common bacterium or fungus as an example, describes a method of drug sensitivity test and a device used therefor. The method was carried out as follows:

a) mixing a target bacterium with 20 mL of a culture solution comprising a colloidal material to yield a liquid test sample;

b) dumping the liquid test sample evenly into a box of a drug sensitivity test device comprising a coagulant aid, the colloidal material of the culture solution reacting with the coagulant aid to yield a stable gel within 3 hrs, thereby a gel test sample obtained;

c) pasting paper comprising to-be-tested drug on the surface of the gel test sample;

d) sealing the box cover using a box cover and culturing in an incubator at 37°C for 12-24 hrs, during the growth and multiplication of the bacterium or fungus, MTT being degraded to yield a blue dot chromophoric group, if the drug inhibiting the growth and multiplication of the bacterium or fungus, no blue dot chromophoric group formed around the gel test sample pasted with paper comprising the drug, and an inhibition zone formed instead; and

e) observing the size of the inhibition zone and determining the effect of the drug on the bacteria.

A formula of 20 mL of the culture solution comprised 20 mL of nutrient broth, 4 mg of 3-(4,5-dimethylthiazol-2-yl)-2,5-diphenyltetrazolium bromide as an indicator, and 0.4 g of 0.5-1.5% sodium alginate as the colloidal material. The coagulant aid was 0.04 g of calcium ion.

The following, taking cancer cells as an example, describes a method of drug sensitivity test and a device used therefor. The method was carried out as follows:

a) separating and culturing a cancer cell collected from a confirmed tumor patient in a solution and storing in a container;

b) mixing 1 mL of the cancer cell solution with 20 mL of a culture solution comprising a colloidal material to yield a liquid test sample;

c) dumping the liquid test sample evenly into a box of a drug sensitivity test device comprising a coagulant aid, the colloidal material of the culture solution reacting with the coagulant aid to yield a stable gel within 3 hrs, thereby a gel test sample obtained;

d) pasting paper comprising to-be-tested drug on the surface of the gel test sample;

e) sealing the box cover using a box cover and culturing in an incubator at 37°C for 2-7 days, during the growth and multiplication of the bacterium or fungus, MTT being degraded to yield a blue dot chromophoric group, if the drug inhibiting the growth and multiplication of the bacterium or fungus, no blue dot chromophoric group formed around the gel test sample pasted with paper comprising the drug, and an inhibition zone formed instead; and

e) observing the size of the inhibition zone and determining the effect of the drug on the bacteria.

A formula of the 20 mL of the culture solution comprised 20 mL of cell culture medium, 5 mg of 3-(4,5-dimethylthiazol-2-yl)-2,5-diphenyltetrazolium bromide as an indicator, and 0.4 g of 0.5-1.5% sodium alginate as the colloidal material. The coagulant aid was 0.04 g of calcium ion.

## Claims

1. A method for testing drug sensitivity of a bacterium or cell, comprising the steps of:
a) concentrating a bacterium or cell sample to be tested;
b) adding a culture solution to the bacterium or cell sample to yield a liquid sample, the culture solution comprising a nutrient and a colloidal material;
c) adding a coagulant aid to the liquid sample where the coagulant aid reacts with the colloidal material to yield a gel test sample;
d) pasting drug paper on the surface of the gel test sample;
e) culturing the gel test sample in an incubator; and
f) observing the formation of a drug inhibition zone and determining the drug sensitivity of the bacterium or cell.

2. The method of claim 1, **characterized in that** the culture solution further comprises a growth indicator of the bacterium or cell.

3. The method of claim 1, **characterized in that** the gel test sample is cultured in the incubator for 2-4 days.

4. The method of claim 2, **characterized in that** the gel test sample is cultured in the incubator for 2-4 days.

5. The method of any of claims 1-4, **characterized in that** the culture solution further comprises a bacteriostat.

6. The method of claim 1, **characterized in that** for each 100 mL of the nutrient, the culture solution comprises 0.8-2 g of the colloidal material and the coagulant aid added thereto is 0.1-0.2 g.

7. The method of any of claims 2-4, **characterized in that** for each 100 mL of the nutrient, the culture solution comprises 0.8-2 g of the colloidal material, the growth indicator of the bacterium or cell added thereto is 2-20 mg, and the coagulant aid 0.1-0.2 g.

8. The method of claim 5, **characterized in that** for each 100 mL of the nutrient, the culture solution comprises 0.8-2 g of the colloidal material, the growth indicator of the bacterium or cell added thereto is 2-20 mg, appropriate bacteriostat, and the coagulant aid 0.1-0.2 g.

9. The method of any of claims 1-4 or 6, **characterized in that** the culture solution is packed and sealed according to a one-time usage amount.

10. The method of claim 5, **characterized in that** the culture solution is packed and sealed according to a one-time usage amount.

11. The method of claim 7, **characterized in that** the culture solution is packed and sealed according to a one-time usage amount.

12. The method of claim 8, **characterized in that** the culture solution is packed and sealed according to a one-time usage amount.

13. A device for testing drug sensitivity of a bacterium or cell according to the method of claim 1, the device comprising a box whose side walls and bottom are enclosed and whose top is open, the box comprising a chamber where a coagulant aid is disposed.

14. The device of claim 13, **characterized in that** a coagulant aid layer is disposed at the bottom of the chamber of the box, comprising a slow-release carrier and the coagulant aid adhered thereto.

15. The device of claim 13, **characterized in that** the coagulant aid layer is disposed at the bottom of the chamber of the box, comprising a mixture of an adhesive and the coagulant aid coated on the bottom of the box.
